# EUROPÄISCHE PATENTSCHRIFT

(11) **EP 1 088 812 B1**
(45) Veröffentlichungstag und Bekanntmachung des Hinweises auf die Patenterteilung: **10.11.2004**
(21) Anmeldenummer: 00119731.8
(22) Anmeldetag: 09.09.2000
(51) Int. Cl.: C07C 49/92, B01J 23/46, B01J 31/22

(54) **Verfahren zur Herstellung von Tris(acetylacetonato)-Iridium-(III)**
Process for the preparation of tris(acetylacetonato)iridium-(III)
Procédé pour la préparation de tris(acétylacétonato)iridium-(III)

(30) Priorität: 28.09.1999 DE 19946545
(43) Veröffentlichungstag der Anmeldung: 04.04.2001
(73) Patentinhaber: W.C. Heraeus GmbH & Co. KG, 63450 Hanau (DE)
(72) Erfinder: Walter, Richard, Dr., 63755 Alzenau (DE); Franz, Renate, 63571 Gelnhausen (DE)
(74) Vertreter: Kühn, Hans-Christian

(56) Entgegenhaltungen:
- US-A- 4 210 608
- US-A- 4 431 574
- DATABASE WPI Week 199440 Derwent Publications Ltd., London, GB; AN 1994-322123 XP002221733 & JP 06 247890 A (SUMITOMO METAL MINING CO), 6. September 1994 (1994-09-06)
- DATABASE WPI Week 199841 Derwent Publications Ltd., London, GB; AN 1998-479655 XP002221734 & RU 2 105 719 C (AS SIBE INORG CHEM INST) , 27. Februar 1998 (1998-02-27)
- J.E. COLLINS ET AL: ORGANOMETALLICS, Bd. 14, Nr. 3, 1995, Seiten 1232-1238, XP002221731
- DATABASE CAPLUS [Online] CHEMICAL ABSTRACTS SERVICE, COLUMBUS, OHIO, US; retrieved from STN Database accession no. 1996:128372 XP002221732 & JP 07 316176 A (TANAKA PRECIOUS METAL IND) 5. Dezember 1995 (1995-12-05)
- F.I. MATTOS-COSTA ET AL: ELECTROCHIMICA ACTA, Bd. 44, Nr. 8-9, 1998, Seiten 1515-1523, XP004144581

## Beschreibung

Die Erfindung betrifft ein Verfahren zur Herstellung von Tris(acetylacetonato)iridium(III) und Verwendungen dieser Verbindung.

Tris(acetylacetonato)iridium(III), im folgenden als Ir-Acetylacetonat bezeichnet, ist aus der Patentliteratur zum Beispiel als Bestandteil einer Lösung zum gleichzeitigen Hydrophobieren und Katalysieren von Kohlenstoffelektroden (DE 15 46 725 A1, Seite 9, Union Carbide Corp.), als Katalysator für die Synthese von Ethylenglykol aus CO und H₂ (US 4,170,606/C07, Spalte 2, Gulf Research and Development) und für die Hydroformylierung und Carbonylierung (EP 786 447/C07, Seite 4) bekannt.

Dwyer et al. beschreiben die Synthese von Iridium-Acetylacetonat (JACS 75, 984 (1953)), bei der zunächst IrO₂ aus K₂IrCl₆ hergestellt, IrO₂ in rauchender Schwefelsäure gelöst, die Säure abgeraucht und ungelöstes IrO₂ abzentrifugiert wird. Anschließend wird Ir(OH)₃ gefällt, die entstandene Suspension mit Acetylacetonat bei einer Temperatur von ca. 60 °C versetzt und schließlich das ausgefallene Iridium-Acetylacetonat abfiltriert.

Nachteilig bei diesem Verfahren ist zum einen der sehr aufwendige Verfahrensablauf sowie die geringe Ausbeute von lediglich 10 %.

Davignon et al.beschreiben die Bildung von Iridium-Acetylacetonat (J. Less Common Metals 21, 1970, 345) nach 3 unterschiedlichen Verfahren.

Nachteilig bei diesen Verfahren ist ebenfalls der sehr aufwendige Verfahrensverlauf und die relativ geringe Ausbeute von lediglich 5 bis 15 %.

Benett et al. beschreiben in Inorganic Chemistry (15 (11), 2936 (1976)) die Synthese von Iridiumacetylacetonat, bei der zunächst IrCl₃ x H₂O/Hacac/Na₂CO₃ 40 Stunden am Rückfluß gekocht und schließlich zum Trocknen eingedampft werden. Anschließend wird mit CH₂Cl₂ extrahiert und der Extrakt mittels Benzol über Aluminiumoxid chromatographiert.

Nachteilig bei diesem zudem aufwendigen Verfahren ist die geringe Ausbeute von lediglich 18 %.

JP 07316176 A (Tanaka) offenbart ein Verfahren zur Herstellung von Iridium-Acetylacetonat bei dem zunächst IrCl₃ in Wasser gelöst wird und anschließend mit Acetylaceton reagiert. Die Lösung wird alkalisch gestellt und das Reaktionsprodukt mittels Benzol extrahiert, eingeengt, gewaschen und getrocknet wird.

Nachteilig bei diesem Verfahren ist eine lediglich 20 %-ige Ausbeute und der Einsatz des relativ teuren IrCl₃-Feststoffs. Darüber hinaus sind die Anforderungen durch die Verwendung von Benzol als Extraktionsmittel relativ hoch und somit kostenungünstig, zumal bei der Extraktion die organische Phase von der wäßrigen Phase getrennt und das Produkt durch Verdampfen des organischen Lösemittels gewonnen werden muß.

Aus dem vorgenannten ergibt sich das Problem, mit Hilfe eines neuartigen Verfahrens die oben genannten Nachteile zumindest teilweise zu beseitigen. Das sich ergebende Problem liegt insbesondere darin, ein einfach durchzuführendes und somit kostengünstiges Verfahren zur Herstellung von Iridium-Acetylacetonat mit einer relativ hohen Ausbeute bereitzustellen.

Dieses Problem wird erfindungsgemäß durch Verfahren nach den Ansprüchen 1 und 2 gelöst.

Bei dem einen erfindungsgemäßen Verfahren wird zunächst eine Iridat(IV)-Lösung zu einer Iridat(III)-Lösung, beispielsweise mittels N₂H₆Cl₂, reduziert. Anschließend wird Acetylaceton und/oder mindestens ein Acetylacetonat-Salz dazugegeben. Die Lösung wird auf einen pH-Wert von ca. 6,5 bis 7,5 eingestellt, auf eine Temperatur von ca. T = + 70 °C und/oder für ca. 48 Stunden erwärmt und schließlich der erhaltene Niederschlag an Iridium-Acetylacetonat separiert, beispielsweise durch Filtration.

Bei dem anderen erfindungsgemäßen Verfahren wird zunächst eine ca. 0,1 molare Iridium (IV)halogenid-Lösung zu einer Iridium(III)halogenid-Lösung, beispielsweise mittels N₂H₆Cl₂, reduziert und anschließend Acetylaceton und/oder mindestens ein Acetylacetonat-Salz hinzugegeben. Die entstandene Lösung wird auf einen pH-Wert von ca. 6,5 bis 7,5 neutralisiert, um anschließend den erhaltenen Niederschlag an Iridiumacetylacetonat zu separieren , beispielsweise mittels Filtrieren.

Bei dem ersten erfindungsgemäßen Verfahren konnte überraschenderweise festgestellt werden, daß das Erwärmen der erhaltenden Lösung auf ca. T = + 70 °C und/oder für ca. 48 Stunden zu einer guten Ausbeute von ca. 22 % führt.

Beim anderen erfindungsgemäßen Verfahren konnte überraschenderweise festgestellt werden, daß die Verwendung einer ca. 0,1 molaren Iridium-Halogenid-Lösung zu einem sehr reinen Endprodukt führt. Höhere Konzentrationen führen überraschenderweise zu erheblich stärker verunreinigten Endprodukten, während geringere Konzentrationen eine schlechtere Ausbeute zur Folge haben.

Bei beiden Verfahren sollten jeweils mindestens dreifach-molare Mengen an Acetylaceton und/oder Acetylacetonat zur reduzierten Iridat(III)-Lösung hinzugegeben werden, um zumindest theoretisch die maximale Ausbeute an Iridium-Acetylacetonat zu ermöglichen.

Die der Reaktionslösung zugesetzte basische Verbindung kann beispielsweise Natron-, Kalilauge oder eine andere basische Verbindung, beispielsweise ein Carbonatsalz, sein.

Von besonderem Vorteil ist es, wenn beim ersten erfindungsgemäßen Verfahren bei der Reduktion eine Ir(IV)-Halogenid-Lösung verwendet wird, da sich diese Substanz in der Praxis bewährt hat. Entsprechendes gilt auch für beide erfindungsgemäße Verfahren, wenn bei der Reduktion eine Hexachloro-iridat-Lösung verwendet wird.

Beim zweiten erfindungsgemäßen Verfahren ist es aus schon oben erwähnten Gründen vorteilhaft, wenn nach der Neutralisation die erhaltene Lösung auf eine Temperatur von ca. T = + 70 °C und/oder für ca. 48 Stunden erwärmt wird.

Auch die Reduktion bei beiden Verfahren mittels N₂H₆Cl₂ hat sich in der Praxis bestens bewährt.

Schließlich ist festzustellen, daß das Separieren des erhaltenden Iridium-Acetylacetonat-Niederschlages extraktionsfrei, beispielsweise mittels Filtrieren, durchgeführt wird, da somit die aufwendigen Verfahrensschritte entfallen.

Das auf diese Weise hergestellte Iridium-Acetylacetonat kann durch seine hohe Reinheit als Katalysator oder Katalysatorvorstufe, insbesondere für Hydroformylierungen, als Ausgangsverbindung für keramische Farben und als Ausgangsverbindung für die Herstellung eines heterogenen Katalysators dienen.

Das nachfolgende Beispiel dient zur Erläuterung der Erfindung.

In einem 500ml-Dreihalskolben mit aufgesetztem Intensivkühler werden 5 g Iridium in Form von H₃IrCl₆-Lösung eingewogen und mit destilliertem Wasser auf ein Volumen von ca. 250 ml aufgefüllt. Dann gibt man unter Rühren 8,5 ml Acetylaceton dazu und neutralisiert die Lösung mit Natriumhydrogencarbonat bis zu einem pH-Wert von 7, wobei Kohlendioxid freigesetzt wird. Die Reaktionsiösung wird 48 Stunden lang bei einer Temperatur von ca. +70 °C gehalten. Dabei fällt Iridium-Acetylacetonat als gelber Feststoff aus, der abfiltriert und mit ca. 300 ml dest. Wasser gewaschen wird. Danach wird der Feststoff bei ca. +75 °C im Vakuumtrockenschrank getrocknet.

Man erhält 2,90 g Iridium-Acetylacetonat (1,14 g Ir) in Form eines gelben Pulvers. Die Ausbeute beträgt 23 %, bezogen auf das eingesetzte Iridium.

Die Identität des Produktes wurde mittels Dünnschichtchromatographie bestätigt.

## Patentansprüche

1. Verfahren zur Herstellung von Tris(acetylacetonato)iridium(III), **gekennzeichnet durch**:
a) Reduktion einer Iridat(IV)-Lösung zu einer Iridat(III)-Lösung,
b) Zugabe von Acetylaceton und/oder mindestens eines Acetylacetonat-Salzes,
c) Neutralisation der Lösung auf einen pH-Wert von ca. 6,5 bis 7,5,
d) Erwärmen der erhaltenen Lösung auf ca. T = + 70 °C und/oder für ca. 48 Stunden,
e) Separieren des erhaltenen Tris(acetylacetonato)iridium(III)-Niederschlags.

2. Verfahren zur Herstellung von Tris(acetylacetonato)iridium(III), **gekennzeichnet durch**:
a) Reduktion einer ca. 0,1 molaren Halogeno-Iridat(IV)-Lösung zu einer Iridat(III)-Lösung,
b) Zugabe von Acetylaceton und/oder mindestens eines Acetylacetonat-Salzes,
c) Neutralisation der Lösung auf einen pH-Wert von ca. 6,5 bis 7,5,
d) Separieren des erhaltenen Tris(acetylacetonato)iridium(III)-Niederschlags.

3. Verfahren nach Anspruch 1, **dadurch gekennzeichnet, dass** beim Schritt a) eine Iridium(IV)-Halogenid-Lösung verwendet wird.

4. Verfahren nach einem der Ansprüche 1 oder 3, **dadurch gekennzeichnet, dass** beim Schritt a) eine Hexachloro-Iridium-(IV)-Lösung verwendet wird.

5. Verfahren nach Anspruch 2, **dadurch gekennzeichnet, dass** beim Schritt a) eine Hexachloro-Iridium-(IV)-Lösung verwendet wird.

6. Verfahren nach Anspruch 2 oder 5, **dadurch gekennzeichnet, dass** nach der Neutralisation die erhaltene Lösung auf ca. T = + 70 °C und/oder für ca. 48 Stunden erwärmt wird.

7. Verfahren nach einem Ansprüche 1 bis 6, **dadurch gekennzeichnet, dass** das Separieren des erhaltenen Tris(acetylacetonato)iridium(III)-Niederschlags extraktionsfrei durchgeführt wird.

8. Verfahren nach einem Ansprüche 1 bis 7, **dadurch gekennzeichnet, dass** die Reduktion mittels N₂H₆Cl₂ durchgeführt wird.

## Claims

1. Process for the preparation of tris(acetylacetonato)iridium(III), **characterized by**:
a) reduction of an iridate(IV) solution to an iridate(III) solution,
b) addition of acetylacetone and/or at least one acetylacetonate salt,
c) neutralization of the solution to a pH of about 6.5 to 7.5,
d) heating of the solution obtained to about T = +70°C and/or for about 48 hours,
e) separation of the tris(acetylacetonato)-iridium(III) precipitate obtained.

2. Process for the preparation of tris(acetylacetonato)iridium(III), **characterized by**:
a) reduction of an approx. 0.1 molar halogenoiridate(IV) solution to an iridate(III) solution,
b) addition of acetylacetone and/or at least one acetylacetonate salt,
c) neutralization of the solution to a pH of approx. 6.5 to 7.5,
d) separation of the tris(acetylacetonato)-iridium(III) precipitate obtained.

3. Process according to Claim 1, **characterized in that** an iridium(IV) halide solution is used in step a).

4. Process according to either of Claims 1 or 3, **characterized in that** a hexachloroiridium(IV) solution is used in step a).

5. Process according to Claim 2, **characterized in that** a hexachloroiridium(IV) solution is used in step a).

6. Process according to Claim 2 or 5, **characterized in that**, after the neutralization, the solution obtained is heated to approx. T = +70°C and/or for about 48 hours.

7. Process according to any of Claims 1 to 6, **characterized in that** the separation of the tris(acetylacetonato)iridium(III) precipitate obtained is carried out without extraction.

8. Process according to any of Claims 1 to 7, **characterized in that** the reduction is carried out by means of N₂H₆Cl₂.

## Revendications

1. Procédé de préparation de tris(acétylacétonato)iridium (III), **caractérisé par** :
a) la réduction d'une solution d'iridate (IV) en une solution d'iridate (III),
b) l'addition d'acétylacétone et/ou d'au moins un sel acétylacétonate,
c) la neutralisation de la solution à un pH d'environ 6,5 à 7,5,
d) le chauffage de la solution obtenue à environ T = + 70°C et/ou pendant environ 48 heures,
e) la séparation du précipité de tris(acétylacétonato)iridium (III) obtenu.

2. Procédé de préparation de tris(acétylacétonato)iridium (III) **caractérisé par** :
a) la réduction d'une solution d'halogéno-iridate (IV) environ 0,1 fois molaire en une solution d'iridate (III),
b) l'addition d'acétylacétone et/ou d'au moins un sel acétylacétonate,
c) la neutralisation de la solution à un pH d'environ 6,5 à 7,5,
d) la séparation du précipité de tris(acétylacétonato)iridium (III) obtenu.

3. Procédé selon la revendication 1 **caractérisé en ce qu'**une solution d'halogénure d'iridium (IV) est utilisée dans l'étape a).

4. Procédé selon l'une des revendications 1 et 3 **caractérisé en ce qu'**une solution d'hexachloro-iridium (IV) est utilisée dans l'étape a).

5. Procédé selon la revendication 2 **caractérisé en ce qu'**une solution d'hexachloro-iridium (IV) est utilisée dans l'étape a).

6. Procédé selon la revendication 2 ou 5 **caractérisé en ce que**, après la neutralisation, la solution obtenue est chauffée à environ T = + 70°C et/ou pendant environ 48 heures.

7. Procédé selon l'une des revendications 1 à 6 **caractérisé en ce que** la séparation du précipité de tris(acétylacétonato)iridium (III) obtenu est réalisée sans extraction.

8. Procédé selon l'une des revendications 1 à 7 **caractérisé en ce que** la réduction est réalisée avec N₂H₆Cl₂.
